(19) Europäisches Patentamt
European Patent Office
Office européen des brevets

(11) Veröffentlichungsnummer: **0 013 891**
**B1**

# (12) EUROPÄISCHE PATENTSCHRIFT

(45) Veröffentlichungstag der Patentschrift: **19.05.82**

(51) Int. Cl.³: **C 07 C 103/52**, A 61 K 37/02

(21) Anmeldenummer: **80100057.1**

(22) Anmeldetag: **07.01.80**

## (54) Dipeptide zur Verwendung bei der Heilung von Krankheiten, einige neue Stoffe aus dieser Verbindungsgruppe, Dipeptide enthaltende Arzneimittel und deren Herstellung.

(30) Priorität: **17.01.79 DE 2901667**

(43) Veröffentlichungstag der Anmeldung: **06.08.80 (Patentblatt 80/16)**

(45) Bekanntmachung des Hinweises auf die Patenterteilung: **19.05.82 Patentblatt 82/20**

(84) Benannte Vertragsstaaten: **AT BE CH DE FR GB IT LU NL SE**

(56) Entgegenhaltungen:
**BIOCHEMISTRY, Band 5, Nr. 7, Juli 1966, Edit. American Chemical Society, K. INOUYE et al.: « New synthetic substrates for Pepsin », Seiten 2 473-2 477**

**JOURNAL OF MEDICINAL CHEMISTRY, Band 11, Nr. 1, 26. Dezember 1967, American Chemical Society, E. NICOLAIDES et al.: « Potential antiviral agents. Carbobenzoxy Di- and Tripeptides active against Measles and Herpes Viruses », Seiten 74-79**

**THE JOURNAL OF ORGANIC CHEMISTRY, Band 28, Nr. 7, Juli 1963, Edit. Amer. Chemic. Society, M. WILCHEK et al.: «The synthesis of Tryptophan peptides», Seiten 1 874-1 875**

(73) Patentinhaber: **Troponwerke GmbH & Co. KG**
**Berliner Strasse 156**
**D-5000 Köln 80 (DE)**

(72) Erfinder: **Jacobi, Haireddin, Dr.**
**Finkenweg 2**
**D-5653 Leichlingen (DE)**
Erfinder: **Opitz, Wolfgang, Dr.**
**Holzbachtalstrasse 60**
**D-5063 Overath (DE)**
Erfinder: **Etschenberg, Eugen, Dr.**
**Virchowstrasse 20**
**D-5000 Köln 41 (DE)**

(74) Vertreter: **Dill, Erwin, Dr. et al**
**c/o BAYER AG Zentralbereich Patente Marken und Lizenzen Bayerwerk**
**D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 013 891 B1

Imprimerie Jouve, 18, rue St-Denis, 75001 Paris, France

Dipeptide zur Verwendung bei der Heilung von Krankheiten, einige neue Stoffe aus dieser Verbindungsgruppe, Dipeptide enthaltende Arzneimittel und deren Herstellung

Die vorliegende Erfindung betrifft Dipeptide zur Verwendung als tumorgewebs- und gewebsauflösende Arzneimittel sowie einige neue Verbindungen aus dieser Verbindungsklasse, diese enthaltende Arzneimittel und deren Herstellung.

Es ist bereits bekannt, daß man eine tumorgewebs- und gewebsauflösende Wirkung mit Stoffen verschiedenster Art erreichen kann. Die allgemeine Toxizität solcher Verbindungen ist jedoch in den meisten Fällen so groß, daß therapeutisch gut handhabbare Behandlungsmöglichkeiten, die den Patienten nicht noch mehr schädigen, nicht existieren.

Es wurde deshalb vorgeschlagen, die wenig toxischen Dehydrooligopeptide (DT-OS 26 59 154) einzusetzen, welche jedoch gegenüber den erfindungsgemäßen Verbindungen einige Nachteile aufweisen.

Einige der erfindungsgemäßen Dipeptide sind bereits bekannt geworden in dem Britischen Patent 1 415 506. Ihre tumorgewebs- und gewebsauflösende Wirkung ist bisher noch nicht bekannt geworden.

Die Erfindung betrifft Dipeptide der allgemeinen Formel I

$$R—Phe—Trp—R_1 \qquad (I)$$

in welcher

Phe für den Rest

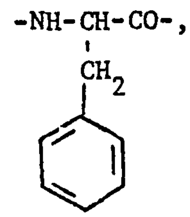

steht,

Trp für den Rest

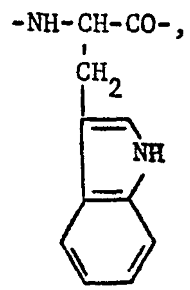

steht,

R für ein Wasserstoffatom oder eine niedere, gegebenenfalls substituierte Alkanoylgruppe steht und

$R_1$ für eine Hydroxy-, niedere Alkoxy-, Amino-, niedere Alkylamino- oder niedere Dialkylaminogruppe steht,

und deren mit Basen und Säuren gebildeten physiologisch verträglichen Salze sowie ihre verschiedenen stereoisomeren Formen zur Verwendung als tumorgewebs- und gewebsauflösende Arzneimittel.

Die vorliegende Erfindung umfaßt ebenfalls die bisher nicht bekannten Verbindungen

H—D—Phe—D—Trp—$NH_2$,
H—D—Phe—D—Trp—OMe,
Acetyl—D—Phe—D—Trp—OH und
Acetyl—D—Phe—D—Trp—$NH_2$.

In der allgemeinen Formel I bedeutet eine gegebenenfalls substituierte Alkanoylgruppe für R insbesondere eine gesättigte gerad- oder verzweigtkettige Alkanoylgruppe mit 1 bis 6 Kohlenstoffatomen. Im einzelnen seien genannt die Formyl-, Acetyl-, Propionyl-, Acryl-, Crotonyl-, Butyryl- und Valerylgruppe, besonders bevorzugt ist die Acetylgruppe. Diese Gruppen sind gegebenenfalls substituiert durch eine Hydroxylgruppe oder durch ein oder mehrere Halogenatome.

Unter einer Alkoxy-, Alkylamino- und Dialkylaminogruppe für $R_1$ sind insbesondere solche zu verstehen, welche im Alkylteil 1 bis 4 Kohlenstoffatome aufweisen. Im einzelnen seien für die Alkylradikale genannt die Methyl-, Äthyl-, Propyl-, Isopropyl- und die verschiedenen gerad- oder verzweigtkettigen

Butylgruppen ; besonders bevorzugt ist die Methylgruppe.

Die Darstellung der erfindungsgemäßen Verbindungen erfolgt in an sich bekannter Weise nach den üblichen, in der Peptidchemie gebräuchlichen Methoden (vgl. Houben-Weyl, Methoden der organischen Chemie, Bd. 15/1 und 15/2).

Beispielhaft sei folgender Reaktionsverlauf angegeben :

Unter Schutz der Aminogruppe mit der tert.-Butoxycarbonylgruppe wird Phenylalanin als Boc—Phe—OH mit dem Methylester von Tryptophan, Trp—OMe · HCl, bei niedriger Temperatur in Gegenwart eines säurebindenden Mittels und eines Kondensationsmittels, z.B. Dicyclohexylcarbodiimid, kondensiert zu Boc—Phe—Trp—OMe, anschließend zunächst die Estergruppierung mit Alkali verseift zu Boc—Phe—Trp—OH und schließlich mit Säure die tert.-Butoxycarbonylgruppe abgespalten (H—Phe—Trp—OH).

Liegt eine der beiden Aminosäuren in der D,L-Form vor, so können die Diastereomerenpaare aufgrund ihrer unterschiedlichen physikalischen Eigenschaften durch Chromatographie an Kieselgel getrennt werden.

Für den Fall, daß in der allgemeinen Formel I R eine Alkanoylgruppe ist, wird die freie Aminogruppe des entsprechenden Dipeptidesters in bekannter Weise acyliert (Helv. Chim. Acta *41* (1958), 1852).

Für den Fall, daß $R_1$ eine Aminogruppe ist, wird der jeweilige Methylester in üblicher Weise durch Umsetzung mit $NH_3$ in das Amid umgewandelt.

Als Beispiele der erfindungsgemäßen Wirkstoffe seien genannt :

H—Phe—Trp—OH,
H—Phe—D—Trp—OH,
H—D—Phe—Trp—OH,
H—D—Phe—D—Trp—OH,
H—D—Phe—D—Trp—OMe,
Ac—D—Phe—D—Trp—OMe,
Ac—D—Phe—D—Trp—OH,
Ac—D—Phe—D—Trp—$NH_2$ und
H—D—Phe—D—Trp—$NH_2$.

Die erfindungsgemäßen Wirkstoffe besitzen eine dosisabhängige tumorgewebs- und sonstige gewebsauflösende Wirkung, vorzugsweise bei lokaler Applikation. Unter lokal seien hier die folgenden Applikationsarten verstanden :

— subkutan, intrakutan, intratumoral, peritumoral, in Form von Externa in Salben, Gelen, Lotionen, Cremes, Injektionslösungen und -suspensionen. Im unmittelbaren Bereich der Applikationsstellen entstehen Nekrosen. Der nekrotische Bereich ist, wenn er nach außen aufbricht, frei von putridem Material selbst über einen längeren Zeitraum, obwohl bei den Versuchstieren Futter, Kot, Sägespäne und anderes mit der offenen Wunde in Berührung kamen.

Das Tumorgewebe kann jedoch auch einschmelzen bei völlig intakter äußerer Haut.

Das nekrotische Gewebe ist von dem ungebenden Gewebe scharf abgegrenzt ; es wirkt makro- und mikroskopisch wie ausgestanzt.

Das allgemeine Verhalten der Versuchstiere wird durch die Größe der Nekrose nicht beeinflußt. Es kommt zu keiner Vergiftung des Gesamtorganismus.

Die $DL_{50}$ der erfindungsgemäßen Verbindungen bewegt sich im Akutversuch bei der intravenösen Injektion an der Ratte in der Größenordnung von 300 mg/kg.

Bei therapeutischem Einsatz bewegen sich die Dosierungen in der Größenordnung von 0,1 bis 100 mg/kg Körpergewicht, vorzugsweise 1 bis 50 mg/kg.

Die erfindungsgemäßen Wirkstoffe können in bekannter Weise in die für ihre Wirkungsart erforderlichen Formulierungen übergeführt werden wie Salben, Pasten, Cremes, Emulsionen, Suspensionen und Injektionslösungen.

Den erfindungsgemäßen Wirkstoffen können in den Formulierungen geeignete Hilfsstoffe beigemischt werden. Als solche gelten inerte, nichttoxische und pharmazeutisch geeignete halbfeste und flüssige Trägerstoffe, Emulgier- und Dispergiermittel. Hierbei soll die therapeutisch wirksame Verbindung jeweils in einer Konzentration von 1 bis 90 Gew.%, vorzugsweise von 5 bis 50 Gew.-%, vorhanden sein.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Die oben aufgeführten pharmazeutischen Zubereitungen können außer den erfindungsgemäßen Wirkstoffen auch weitere pharmazeutische Wirkstoffe enthalten.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten,

zur Behandlung von solchen Geweben im human- und verterinärmedizinischen Bereich, die den Ablauf der normalen biologischen Funktion hindern und stören.

Solche Gewebe sind z.B. :

— gut- und bösartige Tumoren solider und cystischer Natur, vor allem die bei der chirurgischen Intervention einer lokalen Injektion zugänglichen Metastasen sämtlicher Tumorformen.

Darüber hinaus sind die erfindungsgemäßen Verbindungen anwendbar bei fibrosierenden Geweben jeglicher Art, insbesondere bei der Behandlung von Keloiden, Ulcera crura, Verbrennungsgeschwüren, Decubitalulcera sowie Clavi und Onychomykosen, Narbengewebe und zur Therapie und Prophylaxe von Emboli und Thrombosen.

Ebenso können die erfindungsgemäßen Verbindungen eingesetzt werden zur Auflösung von Muttermalen, Warzen, Atheromen und Lipomen sowie zur Beseitigung von tiefen, unter Umständen fistelnden Abszessen.

Zusätzlich können die erfindungsgemäßen Verbindungen eingesetzt werden zur Regeneration von Kavernomen und Tuberkulomen.

Die erfindungsgemäßen Verbindungen sind ebenfalls einsetzbar zur narbenfreien Regeneration der Substanzdefekte bei der Lepra und anderen Haut-, Schleimhaut- bzw. Epitheldefekten verschiedener Genese, vor allem solcher, die durch Infektionen mit Bakterien, Pilzen und Erregern von Tropenkrankheiten wie z.B. die der Leishmaniosen, Framboesia, Pinta u.a. bedingt sind.

Erläuterungen der Abkürzungen :

| | | |
|---|---|---|
| Ac— | Acetyl | $CH_3CO$— |
| Me— | Methyl | $CH_3$— |
| Boc— | tert.-Butoxycarbonyl | 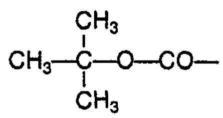 |

## Beispiele

### Beispiel 1 (H—Phe—Trp—OH und H—Phe—D—Trp—OH)

a) Boc—Phe—D,L—Trp—OMe

Zu einer Suspension von 12,5 g (49 mMol) H—D,L—Trp—OMe · HCl in 60 ml Dichlormethan werden bei 0 °C unter Rühren 5,6 g (49 mMol) N-Ethylmorpholin, 13 g (49 mMol) Boc—Phe—OH in 40 ml Dichlormethan und 10,1 g (49 mMol) Dicyclohexylcarbodiimid gegeben. Nach 2-stündigem Rühren bei 0 °C wird der ausgefallene Niederschlag abgesaugt, mit Dichlormethan ausgewaschen und das Filtrat mit 300 ml Ethylacetat verdünnt. Nach mehramligem Auswaschen der Lösung mit gesättigter $KHCO_3$-Lösung, NaCl-Lösung und mit 10 %iger Citronensäure wird über $Na_2SO_4$ getrocknet und im Vakuum eingedampft. Man erhält 22,8 g eines öligen Rohproduktes von Boc—Phe—D,L—Trp—OMe, welches nicht gereinigt wird, da ein Diastereoisomerengemisch mit unterschiedlichen physikalischen Eigenschaften vorliegt.

Dies gilt auch für andere Beispiele.

b) Boc—Phe—D,L—Trp—OH

Zu einer Lösung des vorstehenden Rohproduktes in 80 ml Tetrahydrofuran und 8 ml Wasser werden unter Rühren bei 0 °C 36,8 ml 2nNaOH eingetropft und das Gemisch 1 1/2 Stunden lang bei Raumtemperatur gerührt. Anschließend wird der Ansatz mit 600 ml Wasser verdünnt und mehrmals mit Ethylacetat extrahiert. Die wäßrige Phase wird sodann mit Citronensäure auf pH 2 eingestellt, dreimal mit Ethylacetat extrahiert, der Extrakt über $Na_2SO_4$ getrocknet und im Vakuum eingedampft. Es werden 19,3 g (87,5 % der Theorie) Boc—Phe—D,L—Trp—OH als Rohprodukt erhalten.

c) H—Phe—Trp—OH und H—Phe—D—Trp—OH

Eine Lösung von 9,6 g (21,3 mMol) vorstehenden Rohproduktes in 60 ml 80 %iger Ameisensäure wird 6 Stunden lang bei Raumtemperatur und 45 Minuten lang bei 35 °C unter $N_2$ aufbewahrt, anschließend mit 100 ml Wasser verdünnt und mit Ether extrahiert. Die organische Phase wird mit Wasser extrahiert und die vereinigten wäßrigen Phasen lyophilisiert. Das erhaltene rohe Isomerengemisch wird anschließend grammweise aus Isopropanol/Wasser (5 : 1) an 2 hintereinander geschalteten Fertigsäulen Kieselgel 60, Größe C (Merck, Darmstadt) chromatographiert. Nach Rekristallisation aus Methanol/Wasser werden so erhalten : 1,7 g H—Phe—Trp—OH (22,7 % der Theorie), Fp. 263-266 °C und 1,7 g (22,7 % der Theorie) H—Phe—D—Trp—OH, Fp. 155 °C, $[\alpha]_D^{20} = + 30{,}7°$ (c = 1 ; Methanol) und eine Mischfraktion von 2,6 g (34,6 % der Theorie). Die Ausbeuten sind auf das Diastereomerenpaar als 100 % bezogen.

### Beispiel 2 (H—D—Phe—Trp—OH)

a) Boc—D,L—Phe—Trp—OMe

12,6 g (49,5 mMol) H—Trp—OMe · HCl werden in 80 ml Dimethylformamid gelöst und bei 0 °C unter Rühren mit 7 ml Triethylamin und 22 g (49,5 mMol) Boc—D,L—Phe—OTcp versetzt. Nach 2 1/2 stündigem Stehen wird das Reaktionsgemisch mit 700 ml Wasser verdünnt und 3 mal mit Ethylacetat extrahiert. Der Extrakt wird mit gesättigter $KHCO_3$-Lösung, dann mit 10 %iger Citronensäure und mit gesättigter NaCl-Lösung gewaschen, über $Na_2SO_4$ getrocknet und im Vakuum eingedampft. Nach dem Eindampfen im Vakuum werden 23 g Boc—D,L—Phe—Trp—OMe als Rohprodukt erhalten.

b) Boc—D,L—Phe—Trp—OH

Das vorstehend unter a) beschriebene Rohprodukt wird in 150 ml Tetrahydrofuran und 15 ml wasser gelöst und unter Rühren bei 0 bis 5 °C tropfenweise mit 55 ml 2nNaOH-Lösung versetzt, anschließend wird 1 Stunde lang bei Raumtemperatur gerührt, mit 1,3 l Wasser verdünnt und 2 mal mit Ethylacetat extrahiert. Die wäßrige Phase wird mit konzentrierter Citronensäure-Lösung auf pH 2 eingestellt, 3 × mit Ethylacetat extrahiert, die Extrakte mit gesättigter NaCl-Lösung neutral gewaschen, über $Na_2SO_4$ getrocknet und im Vakuum eingedampft. Es werden 22,4 g Boc—D,L—Phe—Trp—OH als öliges Rohprodukt erhalten.

c) H—Phe—Trp—OH und H—D—Phe—Trp—OH

Das vorstehende unter b) erhaltene Rohprodukt wird in 70 ml 80 %iger Ameisensäure gelöst und 5 1/2 Stunden lang bei Raumtemperatur und über Nacht im Kühlschrank aufbewahrt. Anschließend werden 200 ml Wasser hinzugefügt und die Lösung 2 mal mit Ehter extrahiert. Die wäßrige Phase wird lyophilisiert und das erhaltene Isomerengemisch (12 g, 69 % der Theorie) aus Isopropanol/Wasser an 2 hintereinander geschalteten Fertigsäulen (Kieselgel 60, Größe C, (Merck, Darmstadt)) in Portionen von je 1 g chromatographiert. Bei anschließender Rekristallisation aus Methanol/Wasser werden beide Diastereomeren in reiner Form erhalten.

H—Phe—Trp—OH Fp. 263-265 °C.

$[\alpha]_D^{20} = +14{,}0°$ (c = 0,5 ; nHCl) nach Trocknung bei 50 °C und

$[\alpha]_D^{20} = +16{,}6°$ (c = 0,5 ; nHCl) nach 12-stündiger Trocknung bei 125 °C,

H—D—Phe—Trp—OH Fp. 155 °C.

$[\alpha]_D^{20} = -30{,}6°$ (c = 1 ; Methanol).

Beispiel 3 (H—D—Phe—D—Trp—OH)

a) Boc—D—Phe—D—Trp—OMe

Eine Suspension von 72,8 g (0,286 Mol) H—D—Trp—OMe—HCl in 300 ml Dichlormethan wird bei 0 °C mit 32,9 g (0,286 Mol) N-Ethylmorpholin und anschließend mit 75,9 g (0,286 Mol) Boc—D—Phe—OH, gelöst in 300 ml Dichlormethan, sowie 59 g (0,286 Mol) Dicyclohexylcarbodiimid versetzt und 2 Stundden lang bei 0 °C gerührt. Anschließend wird die Reaktionslösung filtriert, das Filtrat mit 1 l Ethylacetat verdünnt und die erhaltene Lösung mit gesättigter $KHCO_3$-Lösung, 10 %iger Citronensäurelösung und gesättigter NaCl-Lösung gewaschen. Nach dem Trocknen über $Na_2SO_4$ und Eindampfen wird der Trockenrückstand mit Petrolether ausgerührt. Es werden 112,5 g (84,6 % der Theorie) Boc—D—Phe—D—Trp—OMe erhalten. Fp. 164 °C.

$[\alpha]_D^{20} = -11{,}1°$ (c = 0,5 ; Dimethylformamid).

b) Boc—D—Phe—D—Trp—OH

97,8 g (0,21 Mol) Boc—D—Phe—D—Trp—OMe werden wie weiter oben mehrfach beschrieben mit 2 nNaOH verseift und ebenso aufgearbeitet. Es werden 97,6 g rohes Boc—D—Phe—D—Trp—OH isoliert, welches hartnäckig ca. 0,5 Mol Ethylacetat festhält. Eine Probe wird aus Isopropanol/Petrolether rekristallisiert.

Fp. 128-130 °C.

$[\alpha]_D^{20} = -2{,}9°$ (c = 0,5 ; Methanol).

c) H—D—Phe—D—Trp—OH

58,6 g (0,13 Mol) Boc—D—Phe—D—Trp—OH werden in 200 ml einer gesättigten Lösung von HCl in Eisessig eingetragen und unter $N_2$ 1 Stunde lang bei Raumtemperatur aufbewahrt. Nach Versetzen des Reaktionsgemisches mit 400 ml Wasser und Extrahieren mit Ether und Dichlormethan werden die organischen Phasen vereinigt und mit Wasser gewaschen. Die vereinigten wäßrigen Phasen werden lyophilisiert, anschließend in 200 ml heißem Methanol gelöst, filtriert, mit 200 ml Wasser verdünnt und mit konzentrierter $NH_4OH$-Lösung auf pH 5 eingestellt. Die sich abscheidenden Kristalle werden abgesaugt und mit Methanol/Wasser (1 : 2) gewaschen.

Ausbeute : 33,4 g (73,2 % der Theorie) Fp. 281-282 °C.

$[\alpha]_D^{20} = 15{,}5°$ (c = 0,5 ; nHCl).

Beispiel 4 (H—D—Phe—D—Trp—OMe · HCl)

10,6 g (22,8 mMol) Boc—D—Phe—D—Trp—OMe werden in 50 ccm Eisessig (mit HCl gesättigt) gelöst

und 1 Stunde unter Stickstoff bei Raumtemperatur aufbewahrt. Nach Versetzen mit 100 ccm Wasser wird zweimal mit Dichlormethan ausgeschüttelt, die organischen Phasen werden mit Wasser gewaschen, die vereinigten wäßrigen Phasen lyophilisiert und der Trockenrückstand aus 150 ccm Ethanol durch Zugabe von 150 ccm petrolether kristallisiert.

$[\alpha]_D^{20} = -0{,}8°$ (c = 0,5, Methanol).
Ausbeute : 7,4 g (81,3 % der Theorie), Fp. 145 °C.

Beispiel 5 (Ac—D—Phe—D—Trp—OMe)

Eine Suspension von 5,6 g (13,9 mMol) H—D—Phe—D—Trp—OMe·HCl in 400 ccm Dichlormethan wird bei 0 °C zuerst mit 2 ml Triethylamin und anschließend mit 1,64 ml Thioessigsäure versetzt und auf Raumtemperatur erwärmt. Nach 4-stündigem Rühren wird erneut auf 0 °C gekühlt und mit weiteren 1,64 ccm Thioessigsäure versetzt, da noch Ausgangsmaterial vorhanden ist. Man bewahrt das Reaktionsgemisch über Nacht im Kühlschrank auf, filtriert, verdünnt das Filtrat mit Dichlormethan und extrahiert es mit $nNaHCO_3$, nHCl und Wasser. Nach Trocknen der organischen Phase über $Na_2SO_4$ wird im Vakuum zur Trockne eingedampft, der Rückstand mit Diisopropylether ausgerührt und anschließend aus Toluol/Petrolether umkristallisiert.

Ausbeute : 5,45 g (95,5 % der Theorie), Fp. 139-140 °C.
$[\alpha]_D^{20} = -7{,}2°$ (c = 0,5 ; Methanol).

Beispiel 6 (Ac—D—Phe—D—Trp—OH)

Eine Lösung von 2,7 g (6,6 mMol) Ac—D—Phe—D—Trp—OMe in 15 ml Tetrahydrofuran und 1,5 ml Wasser wird bei 0 °C unter Rühren tropfenweise mit 7,3 ml nNaOH versetzt und 1 Stunde bei 0 °C aufbewahrt. Man verdünnt mit 80 ml Wasser, extrahiert neutralstoffe mit Ethylacetat, säuert die wäßrige Phase mit konzentrierter Citronensäure-Lösung an und extrahiert erneut mit Ethylacetat. Die organische Phase wird über $Na_2SO_4$ getrocknet, im Vakuum zur Trockne eingedampft und der Trockenrückstand aus Ethanol/Petrolether (1 : 1) kristallisiert.

Ausbeute : 2,15 g (82,5 % der Theorie), Fp. 228-229 °C.
$[\alpha]_D^{20} = -11{,}8°$ (c = 0,5 ; Dimethylformamid).

Beispiel 7 (Ac—D—Phe—D—Trp—$NH_2$)

Eine Lösung von 2,7 g (6,6 mMol) Ac—D—Phe—D—Trp—OMe in 30 ml Methanol und 3 ml Tetrahydrofuran wird bei 0 °C mit Ammoniak-Gas gesättigt, anschließend 2 Tage bei Raumtemperatur aufbewahrt und dann im Vakuum zur Trockne eingedampft. Der Rückstand wird mit Ether ausgerührt, in heißem Ethanol aufgeschlämmt, die warme Suspension mit Petrolether versetzt und der Niederschlag abgesaugt.

Ausbeute : 2,15 g (82,5 % der Theorie), Fp. 239-241 °C.
$[\alpha]_D^{20} = +20{,}6°$ (c = 0,5 ; Dimethylformamid) ;
berechnet : C 67,33 % H 6,16 % N 14,28 % ;
gefunden : C 67,10 % H 6,07 % N 14,24 %.

Beispiel 8 (H—D—Phe—D—Trp—$NH_2$)

a) Boc—D—Phe—D—Trp—$NH_2$

In eine Suspension aus 46,6 g (0,1 Mol) Boc—D—Phe—D—Trp—OMe in 600 ml Methanol und 60 ml Tetrahydrofuran leitet man unter Rühren bei 0 °C bis zur Sättigung Ammoniakgas (über Natronkalk getrocknet). Nach Stehen über Nacht wird die Lösung filtriert und eingedampft. Der Rückstand wird in 150 ml Aceton heiß gelöst, dann mit 150 ml Petrolether versetzt. Der durch Abkühlen und Anreiben auskristallisierende Festkörper wird abgesaugt.

Ausbeute : 39,6 g (88 % der Theorie), Fp. 176-178 °C.
$[\alpha]_D^{20} = +14{,}6°$ (c = 0,5 ; Dimethylformamid).

b) H—D—Phe—D—Trp—$NH_2$

39,5 g (87,6 mMol) Boc—D—Phe—D—Trp—$NH_2$ werden mit 150 ml Eisessig (mit HCl gesättigt) versetzt und die entstehende Lösung 1 Stunde bei Raumtemperatur stehen gelassen. Der Ansatz wird mit ca. 400 ml Wasser versetzt und 2 mal mit Dichlormethan ausgeschüttelt. Die Dichlormethanlösung wird 1 mal mit Wasser gewaschen. Die vereinigten Wasserphasen werden eingeengt und die restliche Lösung durch Zugabe von konzentrierter ammoniaklösung auf pH 7 bis 8 gebracht. Der durch Anreiben auskristallisierende Festkörper wird abgesaugt und mit Wasser gewaschen. Das Produkt wird in 300 ml warmem Ethanol aufgeschlämmt, mit 300 ml Petrolether versetzt und nach dem Abkühlen abgesaugt.

Ausbeute : 22,5 g (73,3 % der Theorie), Fp. 183 °C.
$[\alpha]_D^{20} = +15{,}6°$ (c = 0,5 ; Dimethylformamid).

**Ansprüche**

1. Dipeptide der allgemeinen Formel I

R—Phe—Trp—$R_1$ (I)

in welcher
Phe für den Rest

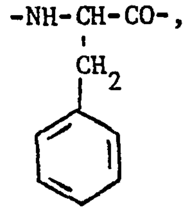

steht,
Trp für den Rest

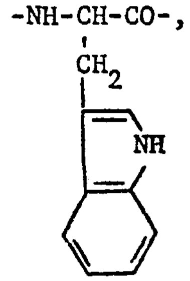

steht,
R für ein Wasserstoffatom oder eine niedere, gegebenenfalls substituierte Alkanoylgruppe steht und
$R_1$ für eine Hydroxy-, niedere Alkoxy-, Amino, niedere Alkylamino- oder niedere Dialkylaminogruppe steht,
und deren mit Basen und Säuren gebildeten physiologisch verträglichen Salze sowie ihre verschiedenen stereoisomeren Formen zur Verwendung als tumorgewebs- und gewebsauflösende Arzneimittel.

2. Dipeptide der allgemeinen Formel I, gemäß Anspruch 1,
in welcher
R für Wasserstoff oder Acetyl steht und
$R_1$ für Hydroxy, Amino, Alkoxy mit 1 bis 2 Kohlenstoffatomen, Alkylamino oder dialkylamino mit je 1 oder 2 Kohlenstoffatomen in den Alkylgruppen, steht,
ihre Salze und ihre optisch isomeren Formen zur Verwendung in tumorgewebs- und gewebsauflösende Arzneimitteln.

3. Dipeptide unter der allgemeinen Formel I gemäß Anspruch 1 der Zusammensetzung

H—D—Phe—D—Trp—$NH_2$,
H—D—Phe—D—Trp—OMe,
Acetyl—D—Phe—D—Trp—OH und
Acetyl—D—Phe—D—Trp—$NH_2$.

4. Tumorgewebs- und gewebsauflösende Arzneimittel gekennzeichnet durch einen Gehalt an mindestens einem Dipeptid der allgemeinen Formel I gemäß Anspruch 1.

5. Arzneimittel gemäß Anspruch 4 enthaltend mindestens ein Dipeptid der allgemeinen Formel I gemäß Anspruch 1,
in welcher
R Wasserstoff beudeutet und
$R_1$ Hydroxy bedeutet.

6. Verfahren zur Herstellung von tumorgewebs- und gewebsauflösenden Arzneimitteln, dadurch gekennzeichnet, daß man Dipeptide der allgemeinen Formel I gemäß Anspruch 1 gegebenenfalls unter Verwendung von inerten nichttoxischen pharmazeutisch geeigneten Trägerstoffen in eine geeigente Applikationsform überführt.

7. Verfahren zur Herstellung von tumorgewebs- und gewebsauflösenden Arzneimitteln gemäß Anspruch 6, dadurch gekennzeichnet, daß man Dipeptide der allgemeinen Formel I gemäß Anspruch 1 unter Verwendung inerter nicht toxischer pharmazeutisch geeigneter Trägerstoffe in eine für lokale Applikation geeignete Form überführt.

**Claims**

1. Dipeptides of the general formula I

R—Phe—Trp—$R_1$ (I)

in which

Phe represents the radical

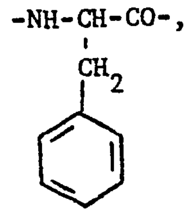

Trp represents the radical

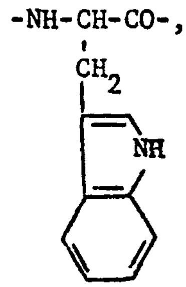

R represents a hydrogen atom or a lower, optionally substituted alkanoyl group

and

$R_1$ represents a hydroxyl, lower alkoxy, amino, lower alkylamino or lower dialkylamino group,

and their physiologically acceptable salts formed with bases and acids, as well as their various stereoisomeric forms for use as tumour tissue-resolving and hystolytic medicaments.

2. Dipeptides of the general formula I, according to Claim 1,

in which

R represents hydrogen or acetyl and

$R_1$ denotes hydroxyl, amino, alkoxy with 1 to 2 carbon atoms or alkylamino or dialkylamino with in each case 1 or 2 carbon atoms in the alkyl groups,

their salt and their optical isomer forms for use in tumour tissue-resolving and histolytic medicaments.

3. Dipeptides of the general formula I according to Claim 1 of the composition :

H—D—Phe—D—Trp—$NH_2$,
H—D—Phe—D—Trp—OMe,
Acetyl—D—Phe—D—Trp—OH and
Acetyl—D—Phe—D—Trp—$NH_2$.

4. Tumour tissue-resolving and histolytic medicaments characterised in that they contain at least one dipeptide of the general formula I according to Claim 1.

5. Medicaments according to Claim 4 containing at least one dipeptide of the general formula I according to Claim 1,

in which

R denotes hydrogen and

$R_1$ denotes hydroxyl.

6. Process for the preparation of tumour tissue-resolving and histolytic medicaments, characterised in that dipeptides of the general formula I according to Claim 1 are converted into a form suitable of administration, inert non-toxic pharmaceutically suitable excipients being used if appropriate.

7. Process for the preparation of tumour tissue-resolving and histolytic medicaments according to Claim 6, characterised in that dipeptides of the general formula I according to Claim 1 are converted into a form suitable for local administration, inert, non-toxic pharmaceutically suitable excipients being used.

**Revendications**

1. Dipeptides de formule générale I

$$R—Phe—Trp—R_1 \quad (I)$$

dans laquelle

Phe représente le reste

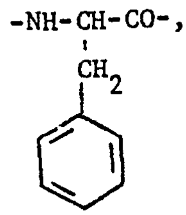

Trp représente le reste

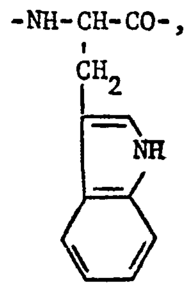

R représente un atome d'hydrogène ou un groupe alcanoyle inférieur éventuellement substitué, et

$R_1$ représente un groupe hydroxy, alcoxy inférieur, amino, alkylamino inférieur ou dialkylamino inférieur,

et leurs sels acceptables pour l'usage pharmaceutique formés avec des bases et des acides, ainsi que leurs diverses formes stéréoisomères, pour l'utilisation en tant que médicaments résolvant les tissus tumoraux et les tissus.

2. Dipeptides de formule générale I selon la revendication 1,

dans laquelle

R représente l'hydrogène ou un groupe acétyle et

$R_1$ représente un groupe hydroxy, amino, alcoxy en $C_1$-$C_2$, alkylamino ou dialkylamino contenant 1 ou 2 atomes de carbone dans chacun des groupes alkyle,

leurs sels et leurs formes énantiomères pour l'utilisation dans des médicaments résolvant les tissus tumoraux et les tissus.

3. Dipeptides de formule générale I selon la revendication 1 et de composition

H—D—Phe—D—Trp—$NH_2$,
H—D—Phe—D—Trp—OMe,
Acetyl—D—Phe—D—Trp—OH et
Acetyl—D—Phe—D—Trp—$NH_2$.

4. Médicament résolvant les tissus tumoraux et les tissus, caractérisé en ce qu'il contient au moins un dipeptide de formule générale I selon la revendication 1.

5. Médicament selon la revendication 4 contenant au moins un dipeptide de formule générale I selon la revendication 1

dans laquelle

R représente l'hydrogène et

$R_1$ représente un groupe hydroxy.

6. Procédé de préparation de médicaments résolvant les tissus tumoraux et les tissus, caractérisé en

ce que l'on met des dipeptides de formule générale I selon la revendication 1 sous une forme d'application appropriée éventuellement avec utilisation de véhicules inertes et non toxiques appropriés à l'usage pharmaceutique.

7. Procédé de préparation de médicaments résolvant les tissus tumoraux et les tissus selon la revendication 6, caractérisé en ce que l'on met des dipeptides de formule générale I selon la revendication 1 sous une forme appropriée à l'application locale par utilisation de véhicules inertes et non toxiques appropriés à l'usage pharmaceutique.